# EUROPEAN PATENT APPLICATION

(11) **EP 2 295 596 A1**
(43) Date of publication of application: **16.03.2011**
(21) Application number: 08748527.2
(22) Date of filing: 23.05.2008
(51) Int. Cl.: C12P 21/06, C07K 1/14, C07K 1/30, C07K 19/00, C12N 15/62

(54) **A METHOD AND KIT FOR PURIFICATION OF RECOMBINANT PROTEINS USING A SELF-CLEAVING PTOTEIN INTEIN**

(71) Applicant: Shantou University, Jinping District Shantou Guangdong 515063 (CN)
(72) Inventor: CHEN, Guoqiang, Guangdong 515063 (CN); WHANG, Zhihui, Guangdong 515063 (CN)
(74) Representative: Daniels, Jeffrey Nicholas
(86) International application number: PCT/CN2008/001006
(87) International publication number: WO 2009/140795

(57) **Abstract**

The present invention provides a method for purifying a recombinant target protein by using an autolytic protein Intein, comprising: recombinantly expressing a fusion protein in a host cell, wherein said fusion protein comprises a target protein domain, an autolytic Intein and one or multiple hydrophobic granule binding domains, and wherein said autolytic Intein is located between said target protein domain and said one or multiple hydrophobic granule binding domains; releasing said fusion protein from said host cell, adding hydrophobic granules and incubating; collecting the incubated hydrophobic granules, adding a lysis solution for allowing the autolytic Intein in said fusion protein to disrupt; and removing said hydrophobic granules, to obtain a solution containing said target proteins which are substantially purified. The present invention further provides a kit for performing such a method.

## Description

### Technical Field

The present invention relates to the purification of a recombinant protein, in particular, to a method and a kit for the purification of a target recombinant protein by using an autolytic protein Intein.

### Technical Background

Nowadays, protein expression techniques have been well developed. Most oligopeptides and polypeptides can be produced. However, these products, such as various enzymes in the biological field and various protein pharmaceuticals for clinical use, usually have to be further purified for further applications. In traditional protein purification procedures, several separation steps by chromatography are needed, and different optimized protocols are required for different proteins. In addition, each step is time-consuming, tedious and relatively expensive. Moreover, multiple steps of separation and purification will largely reduce the yield of the target proteins. The activity of the obtained protein will also be affected to some extent.

To date commercially available affinity purification systems largely simplified the purification steps, however, additional affinity tag segment, e.g., 6 His-tag, has to be added to the target protein. In certain tag-removable purification systems, expensive proteases are required to cleave the affinity adsorption segment from the target protein. However, in such situation, the obtained target protein would be contaminated by the used protease, the separation of which requires other separation steps. In addition, proteases may result in non-specific cleavage(s) within the target protein, causing the target protein to be inactive (Guan C. et al., Gene 67 (1987) 21-30; Martinez et al., Biochem. J. 306 (1995) 589-597).

An autolytic protein known as Intein has been incorporated into IMPACT-TWIN system and IMPACT-CN system produced by NEB (New England Biolabs, 240 County Road, Ipswich, MA 01938-2723, United States). Intein can induce the disruption between itself and the target protein at 25°C, pH 6.0-7.0, or 4°C, pH 8.0-8.5, in the presence of 40 mM mercaptoethanol, thus obtaining the purified protein (Evans T. C. et al., J. Biol. Chem. 274 (1999) 18359-18363; Evans T. C. et al., J. Biol. Chem. 274 (1999) 3923-3926; Mathys S. et al., Gene 231 (1999) 1-13; Southworth M. W. et al., Biotechniques 27 (1999) 110-121.). Such a system overcomes many drawbacks in the traditional protein purification methods, however, it is still not suitable for large-scale production because of its relatively low efficiency and usage of expensive chitin chromatography column.

Polyhydroxyalkanoates (hereinafter abbreviated as PHA) are polymers synthesized by many microorganisms under imbalanced growth conditions (such as deficiency in nitrogen, oxygen, phosphorus, magnesium or the like) for storage of carbon source and energy source (Doi et al., Microbial. 69 (2002) 2498-2504; Anderson et al., Microb. Rev., 54 (1990) 450-472; Lee et al., Biotech. Bioeng., 49 (1996) 1-14). It is present in the cells of various PHA-producing bacteria in the form of insoluble lipid granules. It was reported that PHA granules are coated by a monolayer membrane consisting of PHA synthase (PhaC), PHA depolymerase (PhaZ), granule associated protein (PhaP/phasin), repressor protein (or autoregulator, abbreviated as PhaR), phospholipid monolayer *etc..* (Steinbüchel et al., Can. J. Microbiol. 41 (1995) 94-105; York et al., J. Bacteriol. 183 (1002) 2394-2397; Yamada et al., J. Bacteriol. 189 (2007) 1118-1127).

PHA granules have been successfully produced in many typical strains for protein expression, such as *Escherichia coli* (Fidler et al., FEMS Microbiol. Rev. 9 (1992) 231-235), *Saccharomyces cerevisiae* (Leaf et al., Microbiology 142 (1996) 1169-1180), *Pichia pastoris* (Poirier et al., FEMS Microbiol. Lett. 207 (2002) 97-102) and *etc..* PHA granules will be synthesized, to different extent, once a plasmid containing genes related to PHA-synthesis is transformed into these strains.

Banki *et al.* invents a protein purification system based on PHA granules, which provides new benefits for protein purification and largely contributes to low-cost production of protein purification systems. In this system, both a plasmid containing polyhydroxybutyrate (PHB)-synthesis genes and a plasmid containing PPPI:M (Phasin-Phasin-Phasin-Intein-MBD) (Maltose Binding Domain of Maltose Binding Protein, abbreviated as MBD) are transformed into *E. coli* simultaneously. The expressed fusion protein PPPI:M will bind to PHA granules in the cells due to Phasin's ability to adsorb PHA granules. After ultrasonic disintegration, pure PHA granules will be obtained by sucrose gradient centrifugation. Autolytic protein Intein and MBD will be induced to be disrupted and separated at pH 6.0 and 18-23°C. After another centrifugation, the target protein will be achieved in a high purity from the supernatant (Banki et al., Protein Sci. 14 (2005) 1387-1395; Banki et al., US Publication No. US2006/0141570A1, which are entirely incorporated herein by reference). The main advantage of this method is that it enables the production of purified proteins in a large scale without using expensive affinity columns and proteases, thus largely reducing the expense for purification. However, application of such a method is limited to some extent because of the following reasons: PHA and fusion proteins should be expressed in the same host cell; the binding of the fusion protein and PHA granules occurs within prokaryotic bacterial cells, which requires complicated manipulation; and strains which can produce PHA are limited. In addition, pure PHA granules are obtained by sucrose gradient centrifugation, which is difficult to handle and will increase purification expense. Moreover, protein pharmaceuticals for clinical use are mostly derived from eukaryotic cells, which have to be subject to post-translation modification to be active. However, eukaryotic bacteria which can produce PHA, such as yeasts, produce PHA in a very low yield which is not enough to enable an efficient protein isolation. These factors limit the application of such a system, particularly in development of pharmaceuticals.

PHA depolymerase (PhaZ) contains two functional domains, a substrate binding domain (abbreviated as SBD) and a catalytic domain. Studies show that these two domains are separated by a linker region and function independently (Hisano et al., J. Mol. Biol. 356 (2006) 993-1004). Lee *et al.* fused the SBD of PhaZ with green fluorescent protein EGFP to be expressed, and fixed on PHB microsphere granules (Lee et al., Anal. Chem. 77 (2005) 5755-5759). It suggested that the SBD of PhaZ may be a good adsorption tag.

PhaR, which is a repressor protein of PHA synthesis, can bind to the promoter region of *phaP* gene and *phaR* gene before the formation of PHA granules and thus suppress the expression of both genes. With the gradual formation of PHA granules, the repressor protein PhaR separates from the promoter and then binds to PHA granules (Maehara et al., J. Bacteriol. 184 (2002) 3992-4002). Studies on *phaR* gene mutation show that the repressor protein PhaR also contains two independent functional domains, a DNA binding domain and a substrate binding domain (Yamada et al., J. Bacteriol. 189 (2007) 1118-1127). In addition to PHB surface, PhaR are able to be adsorbed on the surface of polyethylene, polystyrene and polylactic acid (Yamashita et al., Biomacromolecules, 7 (2006) 2449-2454), which demonstrates that the binding between PhaR and PHB is non-specific and is perhaps simply due to hydrophobic interaction.

Therefore, it is still desirable to develop a method for purifying a recombinant protein, which is simple to operate, cost-effective and suitable for large-scale production.

### Summary of the Invention

An object of the present invention is to develop a protein purification system which can operate rapidly, efficiently, cheaply and is suitable for large scale production, thereby overcoming the disadvantages of currently employed protein purification systems, such as involving too many steps, complicated manipulation, high expenses and only suitable for small scale production, and avoiding using expensive materials such as affinity columns and proteases.

Therefore, in an aspect, the present invention provides a method for purifying a recombinant target protein, comprising the steps of:
(1) recombinantly expressing a fusion protein in a host cell, wherein said fusion protein comprises a target protein domain, an autolytic Intein and one or multiple hydrophobic granule binding domains, and wherein said autolytic Intein is located between said target protein domain and said one or multiple hydrophobic granule binding domains;
(2) releasing said fusion protein from said host cell, to obtain a solution containing said fusion protein;
(3) adding hydrophobic granules to said solution and incubating the solution under a condition which allows the binding of said fusion protein with said hydrophobic granules;
(4) collecting the incubated hydrophobic granules from said solution;
(5) adding a lysis solution to said hydrophobic granules and treating said hydrophobic granules under a condition which allows the autolytic Intein in said fusion protein to disrupt; and
(6) removing said hydrophobic granules, to obtain a solution containing said target protein which is substantially purified.

The method may further comprise an optional step of washing said hydrophobic granules after step (4).

In another aspect, the present invention provides a kit for purifying a recombinant target protein, comprising: an expression vector for recombinantly expressing a fusion protein in a host cell, wherein said fusion protein comprises a target protein domain, an autolytic Intein and one or multiple hydrophobic granule binding domains, and wherein the autolytic Intein is located between said target protein domain and said one or multiple hydrophobic granule binding domains; and hydrophobic granules.

The method of the present invention can overcome the disadvantages of the PHA granule-based protein purification system of Banki et *al..* In the method of the present invention, the production of PHA and the expression of the fusion protein are performed independently. Prokaryotic proteins can be expressed in prokaryotic cells while eukaryotic proteins can be expressed in eukaryotic cells; the fusion protein can then bind to hydrophobic granules *in vitro,* which enlarges the range of application of the purification method. In the present invention, hydrophobic granules are made from commonly used hydrophobic materials, such as polystyrene, polyethylene and polylactic acid etc., which are cheap and easily available, thus enlarges the range of application of the purification method.

### Brief Description of the Drawings

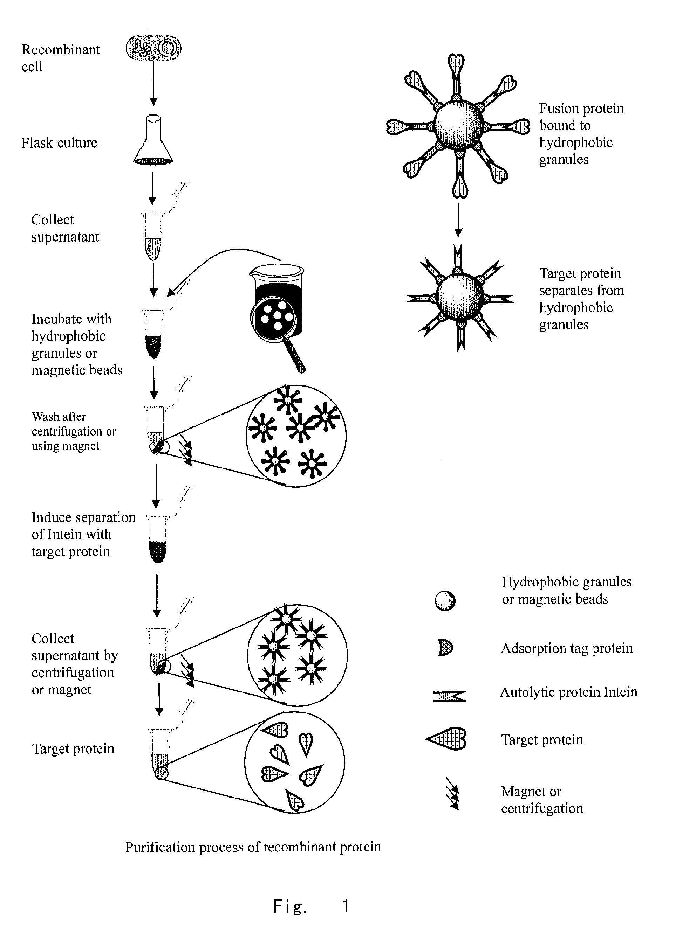

Figure 1 illustrates the procedure of the recombinant protein purification technique by using lipophilic protein as an adsorption tag.

Figure 2 illustrates the plasmid map of a fusion protein prokaryotically expressed in *E. coli.* In the map, "T7 promoter" represents a T7 promoter; "T7 terminator" represents a T7 terminator; "SD sequence" represents a ribosomal binding site (SD sequence); "lac operator" represents the operator gene of lactose operator; "laqI". represents a gene expressing a repressor and "Ssp DnaB intein" represents an intein derived from *Synechocystis* species PCC6803.

Figure 3 illustrates the plasmid map of a fusion protein eukaryotically expressed by *Pichia pastoris.* The word of "origin" represents an origin of replication; "α-factor" represents an α-factor; "ORM1" represents a human α1-acid glycoprotein; and "Mth RIR1 intein" represents an intein derived from *Methanothermobacter thermautotrophicus.*

Figure 4 illustrates the SEM graph for poly(3-hydroxybutyrate-co-3-hydroxyhexanoate) (PHBHHx) hydrophobic granules.

Figure 5 illustrates the plasmid map of pP'PI-EGFP, the hydrophobic granule binding domain of which consists of two different granule associated protein Phasins.

Figure 6 illustrates the SDS-PAGE electrophoresis pattern of green fluorescent protein (EGFP) purified by using the plasmid pPI-EGFP. M: protein molecular weight standard; 1: non-induced whole cells; 2; induced whole cells; 3: the supernatant obtained after cell disruption; 4: the supernatant obtained after binding to the granules; 5: granules with protein bound; 6: the first eluent; 7; the second eluent; 8: granules obtained after Intein disruption; 9: the target protein, EGFP.

Figure 7 illustrates the plasmid map of pPI-lacZ.

Figure 8 illustrates the SDS-PAGE electrophoresis pattern of β-galactidase purified by using the plasmid pPI-lacZ. M: protein molecular weight standard; 1: non-induced whole cells; 2; induced whole cells; 3: the supernatant obtained after cell disruption; 4: the supernatant obtained after binding to the granules; 5: granules with protein bound; 6: the first eluent; 7; the second eluent; 8: granules obtained after Intein disruption; 9: the target protein, β-galactidase.

Figure 9 illustrates the plasmid map of pPI-phaC.

Figure 10 illustrates the SDS-PAGE electrophoresis pattern of PHA synthase (PhaC) purified by using the plasmid pPI-phaC. M: protein molecular weight standard; 1: non-induced whole cells; 2; induced whole cells; 3: the supernatant obtained after cell disruption; 4: the supernatant obtained after binding to the granules; 5: granules with protein bound; 6: the first eluent; 7; the second eluent; 8: granules obtained after Intein disruption; 9: the target protein, PhaC.

Figure 11 illustrates the plasmid map of pRI-EGFP.

Figure 12 illustrates the SDS-PAGE electrophoresis pattern of green fluorescent protein (EGFP) purified by using the plasmid pPI-EGFP. M: protein molecular weight standard; 1: non-induced whole cells; 2; induced whole cells; 3: the supernatant obtained after binding; 4: the first eluent; 5: granules obtained after the first washing; 6: the second eluent; 7: granules obtained after the second washing; 8: the target protein, EGFP; 9: granules obtained after disruption.

Figure 13 illustrates the plasmid map of pSBDI-EGFP.

Figure 14 illustrates the plasmid map of pPI-taq.

Figure 15 illustrates the plasmid map of pPI-malE.

Figure 16 illustrates the SDS-PAGE electrophoresis pattern of maltose binding protein (MBP) purified by using the plasmid pPI-malE. M: protein molecular weight standard; 1: non-induced whole cells; 2; induced whole cells; 3: the supernatant obtained after cell disruption; 4: the supernatant obtained after binding to the granules; 5: granules with protein bound; 6: the eluent; 7: granules obtained after Intein disruption; 8: the target protein, MBP.

Figure 17 illustrates the plasmid map of pPI-GST.

Figure 18 illustrates the plasmid map of pPI-luc.

Figure 19 illustrates the plasmid map of pCI-EGFP wherein PHA synthase (PhaC) was used as an adsorption tag.

Figure 20 illustrates the plasmid map of pPPPI-EGFP, the hydrophobic granule binding domain of which consists of three granule associated protein Phasins.

Figure 21 illustrates the plasmid map of pPSI-EGFP, the hydrophobic granule binding domain of which consists of two different lipophilic proteins, SBD of PhaZ and Phasin.

Figures 22-27 illustrate the nucleotide sequences of SEQ ID Nos: 1-10.

### Detailed Description of the Invention

In an aspect, the present invention provides a method for purifying a recombinant target protein, comprising the steps of:
(1) recombinantly expressing a fusion protein in a host cell, wherein said fusion protein comprises a target protein domain, an autolytic Intein and one or multiple hydrophobic granule binding domains, and wherein said autolytic Intein is located between said target protein domain and said one or multiple hydrophobic granule binding domains;
(2) releasing said fusion protein from said host cell, to obtain a solution containing said fusion protein;
(3) adding hydrophobic granules to said solution and incubating the solution under a condition which allows the binding of said fusion protein with said hydrophobic granules;
(4) collecting the incubated hydrophobic granules from said solution;
(5) adding a lysis solution to said hydrophobic granules and treating said hydrophobic granules under a condition which allows the autolytic Intein in said fusion protein to disrupt; and
(6) removing said hydrophobic granules, to obtain a solution containing said target protein which is substantially purified.

The method may further comprise an optional step of washing said hydrophobic granules after step (4).

Recombinant expression of the fusion protein can be made by introducing an expression vector comprising a nucleic acid encoding said fusion protein into said host cell followed by culturing said host cell. After the expression of the fusion protein, the host cell may be subject to ultrasonic disruption and/or lysozyme treatment, to release the fusion protein from said host cell.

The host cell may be derived from prokaryotes or eukaryotes. Typical prokaryotes suitable for the present invention include but are not limited to *Escherichia coli, Ralstonia eutropha, Pseudomonas* spp., *Bacillus* spp. and *etc..* Typical eukaryotes include but are not limited to *Saccharomyces cerevisiae, Pichia pastoris* and *etc..*

As used herein, the term "target protein" refers to a protein to be obtained by the protein purification method of the present invention. Target proteins particularly suitable for the present invention include, for example, green fluorescent protein, β-galactidase, human α1-acid glycoprotein, taq DNA polymerase, glutathione S-transferase and *etc..* The method of the present invention is particularly suitable for purifying therapeutic proteins, such as human acid fibroblast growth factor aFGF, insulin, interleukin and *etc..*

As used herein, the terms "autolytic Intein", "Intein" and "autolytic protein Intein" can be used interchangeably. They refer to a protein splicing element used for post-translation modification. In protein purification, such an element is often placed between the target protein domain and the affinity tag protein domain. It can be fused and expressed with the target protein and the affinity tag protein. Then the temperature, pH, salt concentration and/or the concentration of mercapto groups can be regulated so as to induce the autolysis of Intein and release the target protein, while the fusion fragment of Intein and the affinity tag protein remain adsorbed on the solid phase medium, thus achieving the purification of the target protein. Usually, commonly used Inteins can autolyze from its N-terminal or C-terminal respectively. Autolytic Inteins suitable for the present invention include but are not limited to Ssp DnaB mini-intein, Mxe GyrA intein, Mth RIR1 intein, Sce VMA1 intein and *etc..*

The autolytic Interin to be employed in the present invention may be an Intein cleavable from the N-terminal of the target protein, such as Ssp DnaB mini-intein (Wu et al., Biochim. Biophys. Acta. 1387 (1998) 422-432); or an Intein cleavable from the C-terminal of the target protein, such as Mxe GyrA intein (Telenti et al., J. Bacteriol. 179 (1997) 6378-6382), Mth RIR1 intein (Smith et al., J. Bioteriol. 179 (1997) 7135-7155) and *etc.,* C-terminal lyzable Intein is sensitive to temperature and pH value. Intein may be effectively disrupted when the temperature is elevated to 25°C or when the pH is reduced from 8.5 to 6.0. Because of the small change of pH value, the occurrence of protein denaturalization caused by the change of pH may also be avoided to some extent (Chong S. et al., Gene 192 (1997)). N-terminal lyzable Intein is more preferred since mercapto compounds are required as an inducer for its disruption, which thus can be controlled effectively. A disruption ratio of 95% can be achieved (Chong S. et al., Nucleic Acids Research, 22 (1998) 5109-5115).

Inteins particularly suitable for the present invention include N-terminal cleavable Ssp DnaB mini-Intein, which can autolyze at pH 6.0-7.0 and/or 18-25°C; C-terminal cleavable Mth RIR1 Intein and Mxe GyrA Intein, which can autolyze at pH 8.5 in the presence of 40 mM mercapto groups. Details for other Inteins can be found in the Intein database of NEB (http://www.neb.com/neb/inteins.html, accessed on May 9, 2008).

The lysis solution to be added for inducing the autolysis of Interin may be water, a buffer solution, or an aqueous solution containing mercapto compounds. A skilled artisan may reasonably select a lysis solution based on the Intein to be used. For example, for Ssp DnaB mini-Intein, a preferred lysis solution is 20mM Tris-Cl, 500mM NaCl and 1 mM EDTA, pH 6.0-7.0. For Mth RIR1 Intein, a preferred lysis solution is 20 mM Tris-HCl, 500 mM NaCl, 1 mM EDTA and 40 mM DTT, pH8.0-8.5.

As used herein, the term "hydrophobic granule binding domain" refers to the part of the fusion protein which is able to closely bind to the hydrophobic granules. The "hydrophobic granule binding domain" in the present invention may be an intact hydrophobic granule binding protein, such as granule associated protein (Phasin/PhaP), PHA synthase (PhaC), PHA depolymerase (PhaZ), repressor protein (PhaR) and lipase; or the substrate binding domain of a hydrophobic granule binding protein, such as substrate binding domain of PHA depolymerase (substrate binding domain of PhaZ, ZSBD) and substrate binding domain of PhaR (RSBD).

The number of the "hydrophobic granule binding domain(s)" of the present invention may be one, two, three or more. Where there are multiple hydrophobic granule binding domains, they may be the same or different to each other, such as Phasin-Phasin, Phasin-Phasin-Phasin, ZSBD-ZSBD-ZSBD, RSBD-RSBD-RSBD, PhaC-PhaC-PhaC, Phasin-ZSBD, ZSBD-RSBD, Phasin-ZSBD-RSBD and *etc.,* Without being bound to any particular theory, the increase in the number of the domains may render their binding to the hydrophobic granules tighter; therby increasing the purity of the obtained target protein.

Preferably, multiple hydrophobic granule binding domains are linked by linker(s). The suitable linker may be any flexible short peptide, which ensures the proper folding of the proteins flanking on both sides into an active form. The examples include linker1 in pTWIN2, Asn Asn Gly Asn Asn Gly Leu Glu Leu Arg Glu Ser Gly (Evans T. C. et al., J. Biol. Chem. 274 (1999) 18359-18363).

Preferably, the autolytic Intein is linked to the one or multiple hydrophobic granule binding domains via a linker. Without being bound to any particular theory, the inventors believe that the use of a linker contributes to ensure the folding of each protein into a proper three-dimensional structure.

Preferably, the autolytic Intein is directly linked to the target protein domain.

In a preferred embodiment of the present invention, the target protein domain, the autolytic Intein and at least one hydrophobic granule binding domain are within the same reading frame.

Preferably, the lipophilic protein, the autolytic protein and the target protein are present as soluble proteins in a microorganism, which usually will simplify the purification process.

As used herein, the term "hydrophobic granule(s)" refers to granules formed by hydrophobic materials and having a hydrophobic surface. The hydrophobic granules of the present invention are not limited to any specific shape and can be spheric, oval, olivary, square or in an irregular shape, as long as it can closely bind to the hydrophobic granule binding domain. The collection and/or isolation of the hydrophobic granules generally can be carried out by centrifugation and/or filtration.

The hydrophobic granules of the present invention may be prepared by emulsion preparation (Sanders et al., J. Am. Chem. Soc. 116 (1993) 2695-2702) or monomer polymerization (Yang et al., Journal of Magnetism and Magnetic Materials 293 (2005) 187-192).

For example, a typical method for preparing hydrophobic granules is as follows:
(1) Oil phase solution: dissolving a hydrophobic polymer material (e.g., PHA, PCL, PMMA and *etc*.) in chloroform at 5% (w/v);
(2) Aqueous phase solution: dissolving a surfactant (e.g., 10 mM sodium oleate, 1% w/v PVA and *etc.*) in water;
(3) pouring the aqueous phase solution into the oil phase solution at the volume ratio of 20:1 (oil/aqueous) and applying ultrasonic treatment to form an emulsion (output power: 50%; working period: 1 second; intermittent period: 1 second; total time period: 10-30 minutes); and
(4) removing the chloroform from the emulsion by a rotation evaporator, achieving the desired emulsion of hydrophobic granules, which are stored at room temperature; and before use, centrifuging the emulsion and collecting the precipitated granules.

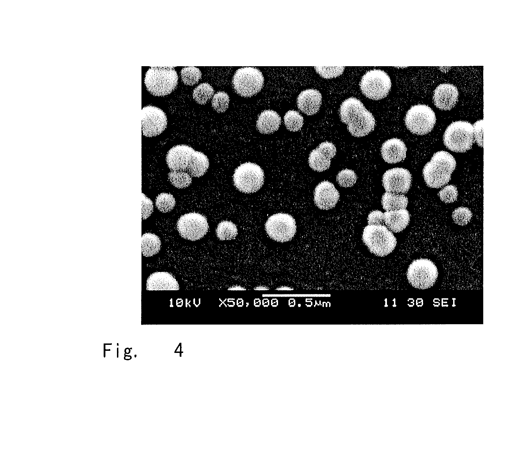

For example, Figure 4 illustrates the SEM graph for poly(3-hydroxybutyrate-co-3-hydroxyhexanoate) (PHBHHx) hydrophobic granules which are particularly preferred in the present invention.

There is no specific requirement to the size of the hydrophobic granules of the present invention. In principle, the smaller the size of the granule, the larger the contact surface to protein will be and thus the higher the amount of the loaded protein per unit granule weight will be, in the mean time the difficulty in centrifugation for collecting the granules might also increase.

The material used to prepare the hydrophobic granules of the present invention may be a hydrophobic polymer material with good liposolubility or a hydrophobic polymer material capable of forming micro-granules via monomer polymerization. Suitable particular hydrophobic materials include: hydrophobic polymer materials such as polyethylene, polyvinyl alcohol, polystyrene, polylactic acid, polycaprolactone, polypropylene, polymethyl methacrylate, polyvinyl chloride and *etc.,* especailly hydrophobic PHA materials such as polyhydroxybutyrate, co-polymer of hydroxybutric acid and hydroxyvaleric acid, co-polymer of hydroxybutric acid and hydroxyhexanoic acid, polyhydroxycaprylate, polyhydroxyenanthate, polyhydroxydecanoate, co-polymer of hydroxybutric acid and hydroxycaprylic acid, co-polymer of hydroxybutric acid, hydroxyvaleric acid and hydroxyhexanoic acid. Polyhydroxybutyrate and co-polymer of hydroxybutric acid and hydroxyvaleric acid are particularly preferred hydrophobic PHA materials.

In a particularly preferred embodiment of the present invention, a magnetic granule is enwraped within the hydrophobic granule. In such a case, a magnet may be conveniently used to collect and/or isolate the hydrophobic granules, which simplifies the purification process. In a preferred embodiment, the hydrophobic granule comprises a core consisting of a super-paramagnetic powder, which core is coated by a hydrophobic material. In a particularly preferred embodiment, the super-paramagnetic powder is Fe₃O₄ magnetic powder. The magnetic powder in the present invention has a high magnetic induction coefficient.

In a particularly preferred embodiment of the present invention, the binding solution comprises Tris-Cl, 10 mM, pH 8.5; and the washing solution comprises Tris-CL, 10 mM, pH 6.5.

As used herein, the term "optional" refers to "may have or may not have", "non-essential" or the like. An "optional washing step" means that the washing step may be included or not included, which may be determined by a skilled artisan.

As used herein, the term "substantially purified" target protein means that a skilled artisan can ascertain, by routine assays and based on commonly recognized standards in the field of molecular biology, that the target protein is pure. For example, if chromatography such as HPLC is used for analyzing the purity of the target protein, the purity of the protein is more than 90%, preferably, more than 95%; or if SDS-PAGE is used for analyzing the purity of the target protein, band of contaminating protein is almost invisible, which may, based on common standard in the field of molecular biology, be regarded to be absent.

The recombinant protein of the present invention can be purified by the following representative procedure:
1) expressing the fusion protein of the present invention which contains the target protein in a prokaryotic expression system such as *E. coli* or a eukaryotic expression system such as *Pichia pastoris;*
2) lysing the cells by a lysozyme or ultrasonic treatment and collecting the supernatant by centrifugation;
3) incubating the supernatant with hydrophobic granules or hydrophobic granules with magnetic granules enwraped therein, to allow the fusion protein to bind to the surface of the hydrophobic granules;
4) collecting the hydrophobic granules by centrifugation or using a magnet, and washing the granules for several times;
5) adding a lysis solution and incubating for a suitable period of time; and
6) precipitating the granules by centrifugation or using a magnet and collecting the supernatant, which is a solution of the target protein with a high purity.

In another aspect, the present invention provides a kit for purifying a recombinant target protein, comprising: an expression vector for recombinantly expressing a fusion protein in a host cell, wherein said fusion protein comprises a target protein domain, an autolytic Intein and one or multiple hydrophobic granule binding domains, and wherein the autolytic Intein is located between said target protein domain and said one or multiple hydrophobic granule binding domains; and hydrophobic granules.

The kit may further comprise a container for holding the lysis solution. In case that a magnetic granule is enwraped within the hydrophobic granules, the kit may further comprise a magnet, for collecting and/or isolating the hydrophobic granules.

The present invention will be illustrated in more details with reference to the following examples, which are presented only for illustrative purpose and are not intended to limit the scope of the present invention in any way.

The strains and plasmids used in the experiments are listed in Table 1. All the restriction endonucleases were purchased from NEB Co. Ltd.. DNA polymerase, 2×Pfu Master Mix, were purchased from Beijing Tiangen Biotech Co., Ltd., which was used to clone gene fragments with a size of less than 4 kb. PrimeSTAR HS DNA Polymerase, which was used to clone long gene fragments, was purchased from Takara Co. Ltd.. DNA ligation Ver. 2.0 was purchased from Takara Co. Ltd.. Other biochemical reagents were purchased from Shanghai Bioengineering Co. Ltd.. Description of polymerase chain reaction, DNA ligation, enzyme cleavage and other methods for gene manipulation can be found in Molecular Cloning: A Laboratory Manual (Sambrook et al., 1989).

### Example 1 Preparation of a fusion protein of granule associated protein Phasin, autolytic protein Intein and green fluorescent protein EGFP as well as the purification of EGFP

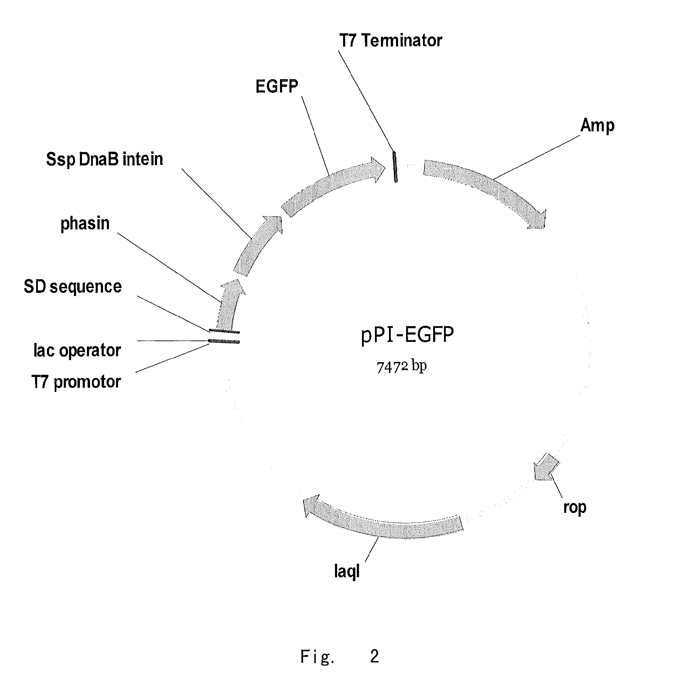

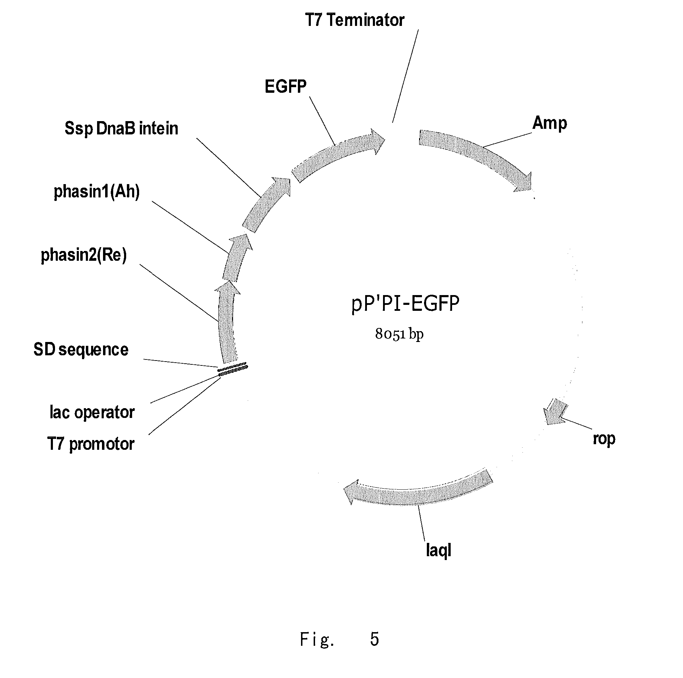

### I. Construction of fusion expression plasmids pPI-EGFP (Figure 2) and pP'PI-EGFP (Figure 5), for expressing the fusion protein phasin-intein-EGFP and the fusion protein phasin'-phasin-intein-EGFP respectively

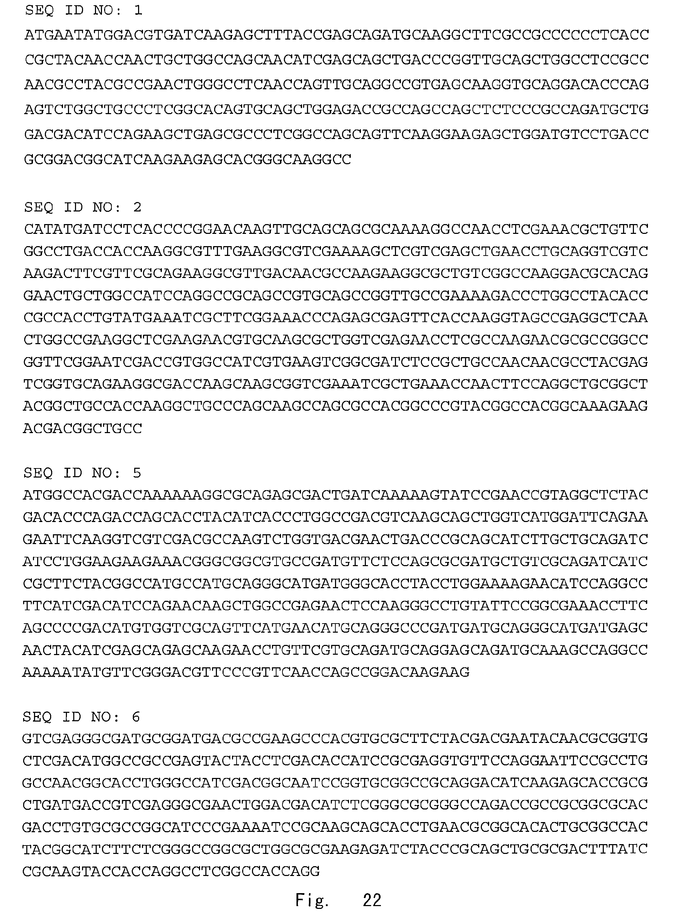

Desired expression plamids were constructed based on modification of plasmid pTWIN1. For constructing pPI-EGFP, firstly PCR was performed to obtain the expression gene *phaP*1 (SEQ ID No: 1, see Figure 22) encoding the granule associated protein phasin. The *phaP*1 was obtained by PCR (PCR conditions: 94°C for 3 minutes; 30 cycles of (94°C for 30s, 55°C for 30s and 72°C for 22s); 72°C for 5 minutes and holding at 4°C), using the genome of *Aeromonas hydrophila* 4AK4 as the template. Then fragments in pTWIN plasmid excluding CBD1 was obtained by PCR (PCR conditions: 94°C for 3 minutes; 30 cycles of (94°C for 30s, 55°C for 30s and 72°C for 7 minutes and 10 seconds); 72°C for 5 minutes and holding at 4°C). The obtained *phaP*1 fragment and the pTWIN plasmid excluding CBD1 were blunt-ligated without any further modification. Then a green fluorescent protein gene EGFP was inserted between *Xho*I and *Bam*HI restriction sites. EGFP was obtained by PCR (PCR conditions: 94°C for 3 minutes; 30 cycles of (94°C for 30s, 55°C for 30s and 72°C for 45s); 72°C for 5 minutes and holding at 4°C), using the plasmid pEGFP-N1 as the template. For constructing the plasmid pP'PI-EGFP, the expression gene *phaP*2 (SEQ ID No: 2, see Figure 22) of another granule associated protein phasin' was inserted into the *Nde*I site of the plasmid pPI-EGFP. The *phaP*2 was obtained by PCR (PCR conditions: 94°C for 3 minutes; 30 cycles of (94°C for 30s, 55°C for 30s and 72°C for 35s); 72°C for 5 minutes and holding at 4°C), using the genome of *Ralstonia eutropha* H16 as the template. The final constructed plasmids were sent to Invitrogen Co. Ltd. for sequencing to confirm the proper sequences. For convenient gene manipulation, corresponding enzyme cleavage sites were incorporated into PCR primers. See Table 2 for the used primers.

### II. Expression of protein

1. The constructed expression plasmids were transformed into *E. coli* BL21 (DE3). A single colony was picked into 20 ml LB medium (Amp, 100 µg/ml), which was incubated overnight at 37°C, achieving a seed liquid.

2. Two milliliters (2 ml) of the seed liquid was added into 200 ml LB medium (Amp, 100 µg/ml) at an inoculation ratio of 1%. The medium was incubated at 37°C until an OD₆₀₀ of 0.4-0.6. IPTG was added to a final concentration of 0.5 mM. Induction was carried out overnight at 15°C.

### III. Cell collection and disruption

1. Cells were collected by centrifugation and washed once by using 20 ml distilled water. Then cells were collected by another centrifugation.

2. Twenty milliliters (20 ml) of binding solution (Tris-CL, 10 mM, pH 8.5) were added. The obtained liquid was mixed thoroughly and kept on ice.

3. Ultrasonic disruption was applied (output power: 50%; working period: 1 second; intermittent period: 2 second; total time period: about 10 minutes, until the cells appeared to be semi-transparent).

4. The supernatant was collected by centrifugation (11000 rpm, 4°C, 30min) and stored at -20°C.

### IV. Binding experiment

1. Twenty milliliters (20 ml) of PHBHHx hydrophobic granule emulsion were centrifuged at 11000 rpm for 10 minuets. The granules were collected.

2. Two milliliters (2 ml) of the protein supernatant were added and blown by a pipette tip. The granules were re-suspended and might be vortex-mixed slightly.

3. The suspension was kept at 4°C for 1-4 hours.

### V. Washing and lysis

1. The bound granules were washed twice by 10 ml binding solution (Tris-CL, 10 mM, pH 8.5). It is to be noted that granules should be blown by a pipette and then vortex-mixed slightly.

2. The granules were washed once by 2 ml lysis solution (Tris-CL, 10 mM, pH 6.5).

3. One milliliter (1 ml) of lysis solution was added and mixed by a pipette. The obtained liquid was kept at 25°C for 12-36 hours for inducing Intein disruption.

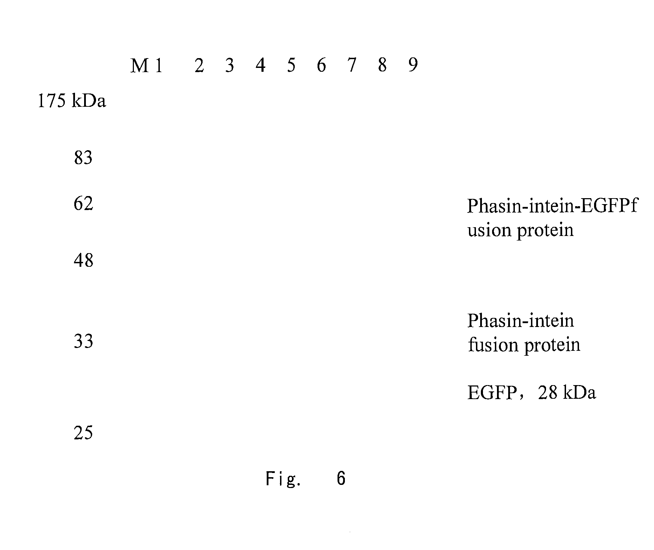

4. The supernatant was collected by centrifugation. The obtained supernatant was the solution of the target protein with a high purity. See Figure 6 for the SDS-PAGE electrophoresis pattern.

Results showed that target protein EGFP was obtained in a high purity.

### Example 2 Preparation of a fusion protein of granule associated protein Phasin, autolytic protein Intein and β-galactosidase (β-gal) as well as the purification of β-galactosidase

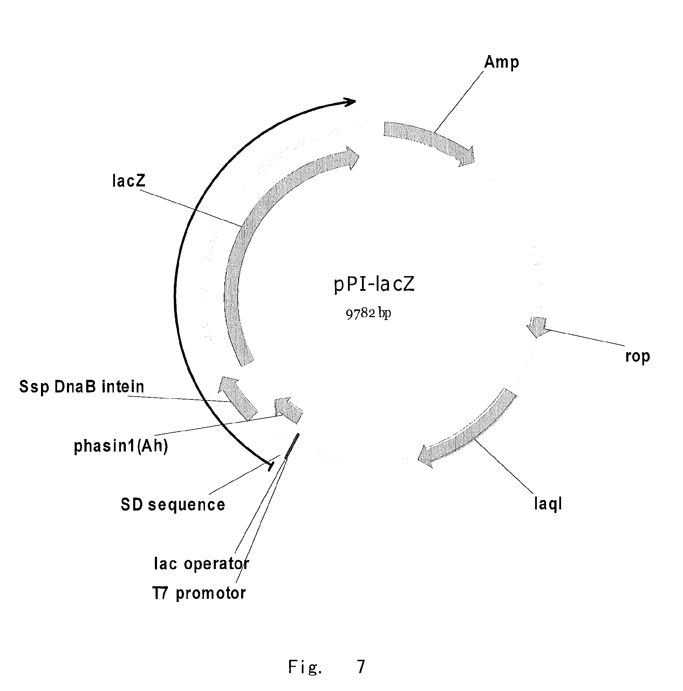

### I. Construction of the fusion expression plasmid pPI-lacZ (Figure 7), for expressing the fusion protein phasin-intein-β-gal

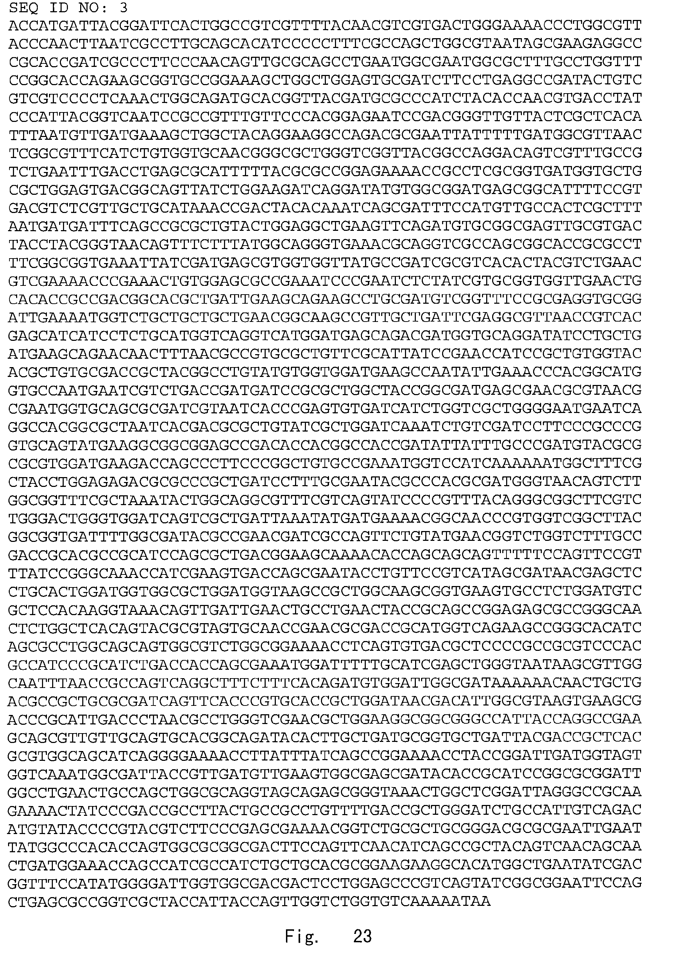

The *lacZ* gene (SEQ ID No: 3, see Figure 23) encoding β-galactosidase was obtained by PCR (PCR conditions: 94°C for 3 minutes; 30 cycles of (94°C for 30s, 55°C for 30s and 72°C for 3 minutes and 10 seconds); 72°C for 5 minutes and holding at 4°C), using the genome of *E. coli* S17-1 as the template. Then the gene was inserted into the *Bsr*GI*lBam*HI site of the plasmid pPI-EGFP. See Table 2 for the used primers.

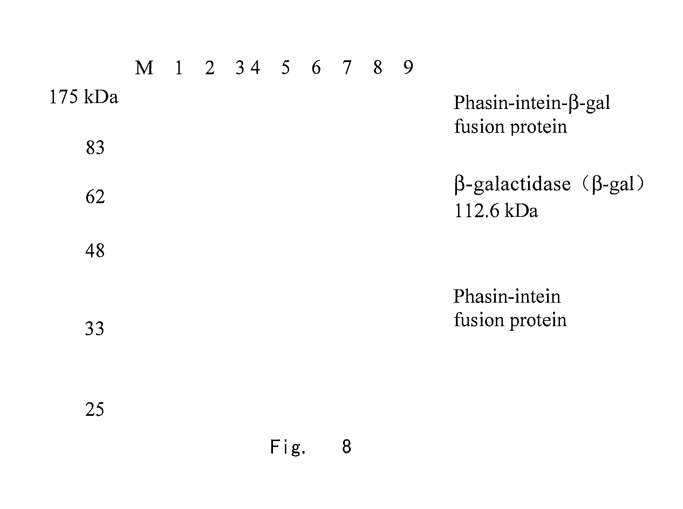

### II. Expression and purification steps were the same as Steps II to V in Example 1. The used hydrophobic granules were made from polylactic acid (PLA). See Figure 8 for the SDS-PAGE electrophoresis pattern.

Results showed that the target protein β-galactosidase was obtained in a high purity.

### Example 3 Preparation of a fusion protein of granule associated protein Phasin, autolytic protein Intein and PHA synthase PhaC as well as the purification of PhaC

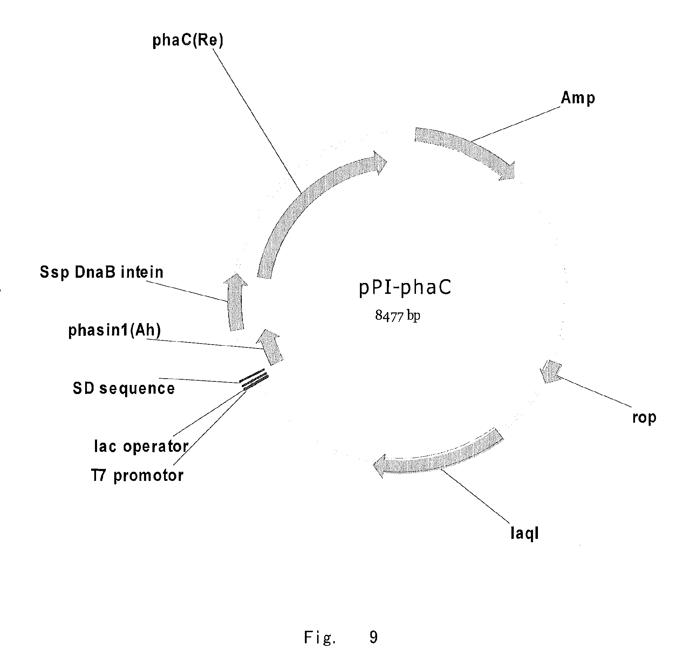

### I. Construction of the fusion expression plasmid pPI-phaC (Figure 9), for expressing the fusion protein phasin-intein-PhaC

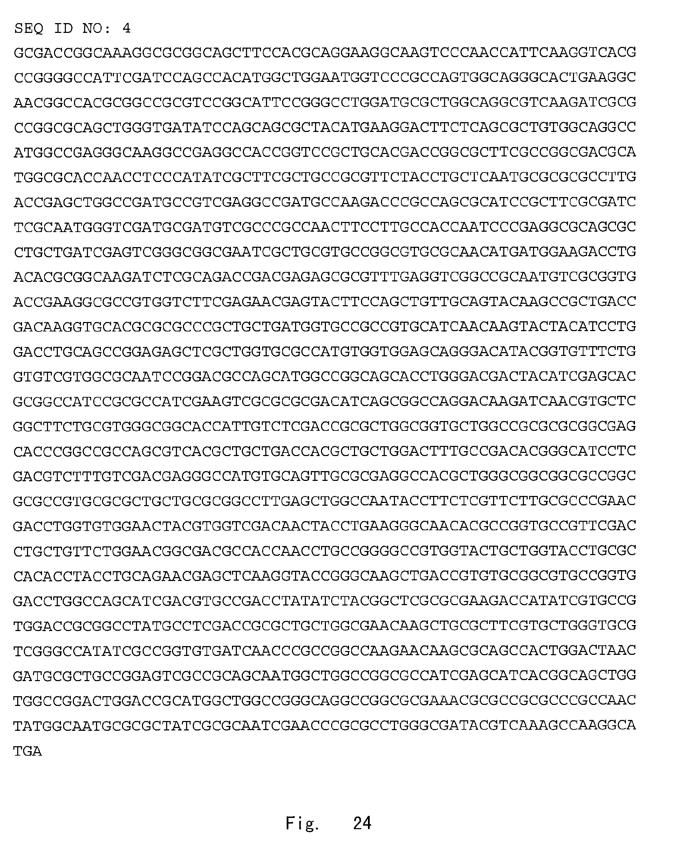

The *phaC* gene (SEQ ID No: 4, see Figure 24) was obtained by PCR (PCR conditions: 94°C for 3 minutes; 30 cycles of (94°C for 30s, 55°C for 30s and 72°C for 1 minute and 50 seconds); 72°C for 5 minutes and holding at 4°C), using the genome of *Ralstonia eutropha* H16 as the template. Then the gene was inserted into the *Bsr*GI*lBam*HI site of the plasmid pPI-EGFP. See Table 2 for the used primers.

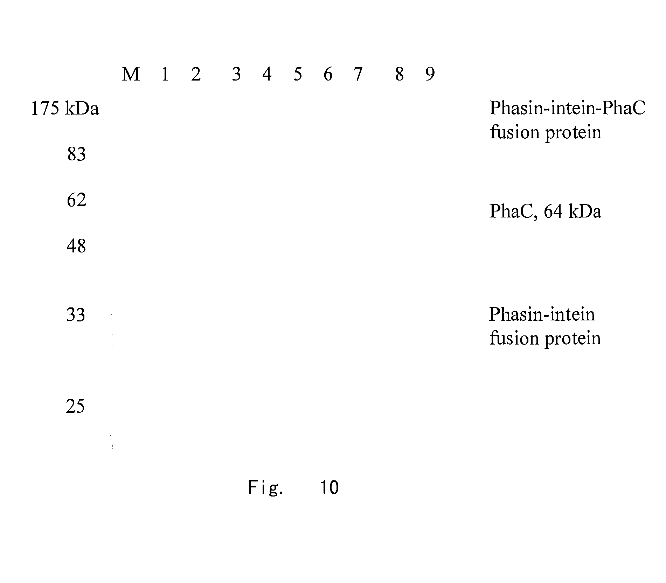

### II. Expression and purification steps were the same as Steps II to V in Example 1. The used hydrophobic granules were made from poly-3-hydroxybutyrate-co-3-hydroxyvalerate (PHBV). See Figure 10 for the SDS-PAGE electrophoresis pattern.

PHA synthase PhaC also has a strong granule binding ability and all the PhaC obtained after the induction of Intein disruption bound to the surface of the hydrophobic granules. Thus no purified target protein PhaC was obtained.

### Example 4 Preparation of a fusion protein of repressor protein PhaR, autolytic protein Intein and green fluorescent protein EGFP as well as the purification of EGFP

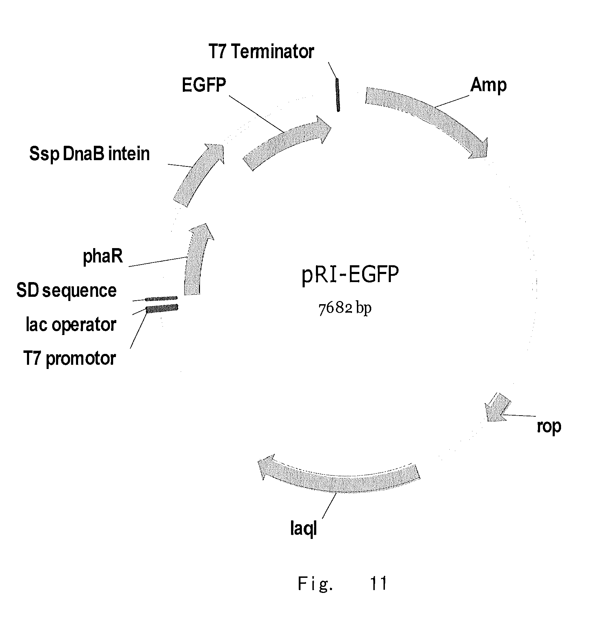

### I. Construction of the fusion expression plasmid pRI-EGFP (Figure 11), for expressing the fusion protein PhaR-intein-EGFP

The *phaR* gene (SEQ ID No: 5, see Figure 22) was obtained by PCR (PCR conditions: 94°C for 3 minutes; 30 cycles of (94°C for 30s, 55°C for 30s and 72°C for 25s); 72°C for 5 minutes, and holding at 4°C), using the genome of *Ralstonia eutropha* H16 as the template. Fragment IE excluding phasin was amplified by PCR (PCR conditions: 98°C for 2 minutes; 30 cycles of (98°C for 10s, 55°C for 10s and 72°C for 7 minutes and 10 seconds); 72°C for 5 minutes and holding at 4°C), using the plasmid pPI-EGFP as the template. Then *phaR* and IE fragments were ligated by *Bgl*II and *Hind*III double enzyme cleavage sites. See Table 2 for the used primers.

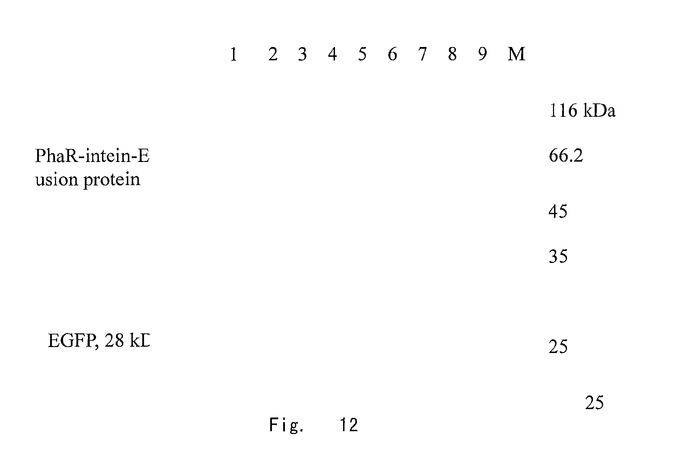

### II. Expression and purification steps were the same as Steps II to V in Example 1. The used hydrophobic granules were made from poly-3-hydroxybutyrate (PHB). See Figure 12 for the SDS-PAGE electrophoresis pattern.

Results showed that the target protein EGFP was obtained in a high purity.

### Example 5 Preparation of a fusion protein of the substrate binding domain of PHA depolymerase PhaZ (ZSBD), autolytic protein Intein and the target protein EGFP

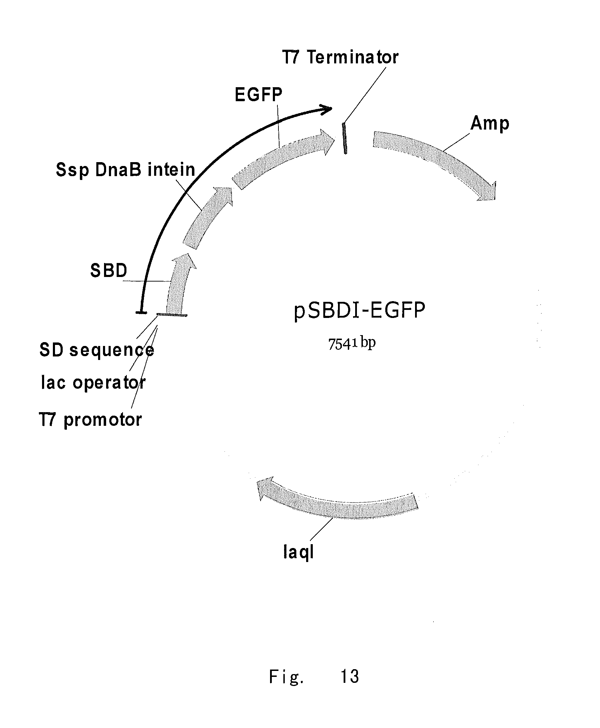

### I. Construction of the fusion expression plasmid pSBDI-EGFP (Figure 13), for expressing the fusion protein ZSBD-intein-EGFP

The ZSBD gene (SEQ ID No: 6, see Figure 22) was obtained by PCR (PCR conditions: 94°C for 3 minutes; 30 cycles of (94°C for 30s, 55°C for 30s and 72°C for 20s); 72°C for 5 minutes and holding at 4°C), using the genome of *Ralstonia eutropha* H16 as the template. Fragment IE excluding phasin was amplified by PCR (PCR conditions: 98°C for 2 minutes; 30 cycles of (98°C for 10s, 55°C for 10s and 72°C for 7 minutes and 10 seconds); 72°C for 5 minutes and holding at 4°C), using the plasmid pPI-EGFP as the template. Then ZSBD and IE fragments were ligated by *Bgl*II and *Hind*III double enzyme cleavage sites. See Table 2 for the used primers.

### II. Expression and purification steps were the same as Steps II to V in Example 1. The used hydrophobic granules were made from polycaprolactone (PCL).

Results showed that the target protein EGFP was obtained in a high purity.

### Example 6 Preparation of a fusion protein of granule associated protein Phasin, autolytic protein Intein and human α1-acid glycoprotein (ORM1) for expression in a eukaryotic expression system Pichia pastoris G115 as well as the purification of ORM1

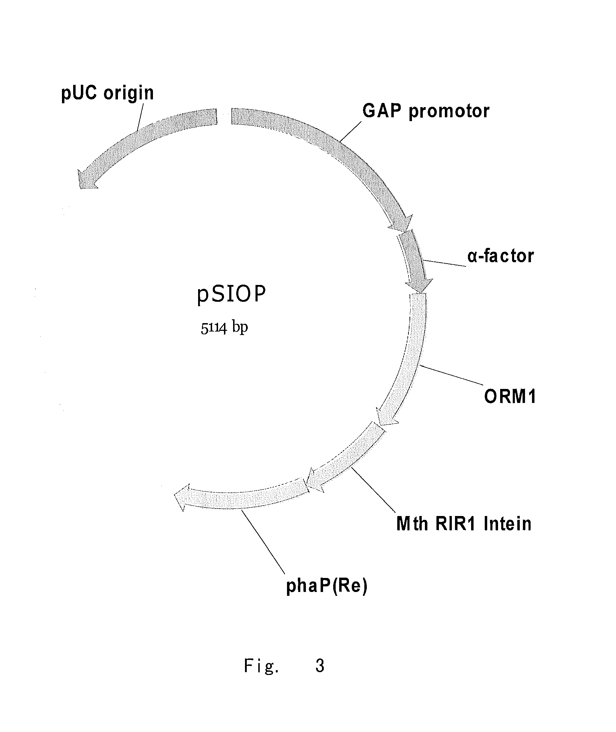

### I. Construction of the yeast expression plasmid pSIOP (Figure 3), for expressing the fusion protein ORM1-intein-phasin

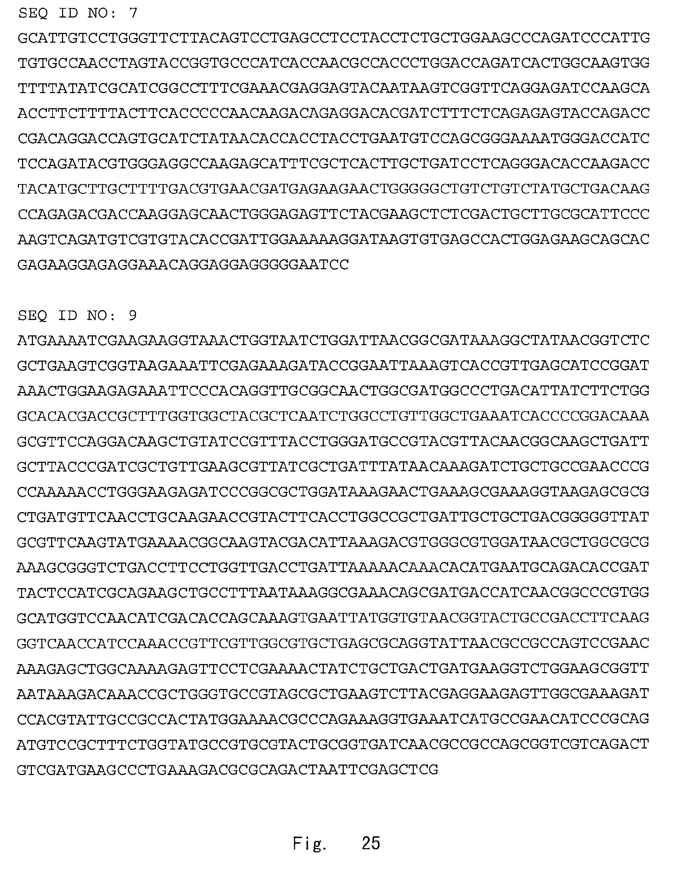

Modification was made on the basis of plasmid pGAPZαA. Firstly, the *phaP* gene (SEQ ID No: 2) was inserted into *Kpn*I*lXba*I double enzyme cleavage sites. The ORM1 gene (SEQ ID No: 7, see Figure 25) was inserted into *Nsi*I/*Kpn*I double enzyme cleavage sites. Then the Intein gene was inserted into *Kpn*I enzyme cleavage site. The obtained plasmid was pSIOP. The *phaP* gene was obtained by PCR (PCR conditions: 94°C for 3 minutes; 30 cycles of (94°C for 30s, 55°C for 30s and 72°C for 35s); 72°C for 5 minutes and holding at 4°C), using the genome of *Ralstonia eutropha* H16 as the template. The Intein gene was obtained by PCR (PCR conditions: 94°C for 3 minutes; 30 cycles of (94°C for 30s, 55°C for 30s and 72°C for 30s); 72°C for 5 minutes and holding at 4°C), using pTWIN1 as the template. The human α1-acid glycoprotein (ORM1) gene was obtained from human liver cDNA library by PCR (PCR conditions: 94°C for 3 minutes; 30 cycles of (94°C for 30s, 55°C for 30s and 72°C for 36s); 72°C for 5 minutes and holding at 4°C). See Table 2 for the used primers.

### II. Expression of protein

1. The constructed expression plasmid was electro-transformed into *Pichia pastoris* GS 115, which was then plated on YPDS medium plate containing 100 µg/ml Zeocin. A single colony was picked into 20 ml YPD medium (Zeocin, 100 µg/ml), which was incubated at 30°C until OD600 reached about 1, thereby obtaining a seed liquid.

2. Two milliliters (2 ml) of the seed liquid was added into 200 ml YPD medium at an inoculation ratio of 1%. The medium was incubated at 30°C for 2-3 days.

### III. Binding experiment

1. The supernatant was collected by centrifugation (11000 rpm, 4°C, 30min) and then stored at -20°C.

2. Twenty milliliters (20 ml) of PHB hydrophobic granule emulsion were centrifuged at 11000 rpm for 10 minuets. The granules were collected.

3. Two milliliters (2 ml) of the protein supernatant were added and blown by a pipette tip. The granules were re-suspended and might be vortex-mixed slightly.

4. The suspension was kept at 4°C for 1-4 hours.

### IV. Washing and lysis

1. The bound granules were washed twice by 10 ml binding solution. It is to be noted that granules should be blown by a pipette and then vortex-mixed slightly.

2. The granules were re-washed once by 2 ml lysis solution (Tris-CL, 10 mM, pH 6.5).

3. One milliliter (1 ml) of lysis solution was added and mixed by a pipette. The obtained liquid was kept at 25°C for 12-36 hours for inducing Intein disruption.

4. The supernatant was collected by centrifugation. The obtained supernatant was the solution of the target protein with a high purity.

Results showed that the target protein ORM1 was obtained in a high purity.

### Example 7 Preparation of a fusion protein of granule associated protein Phasin, autolytic protein Intein and taq DNA polymerase as well as the purification of taq DNA polymerase

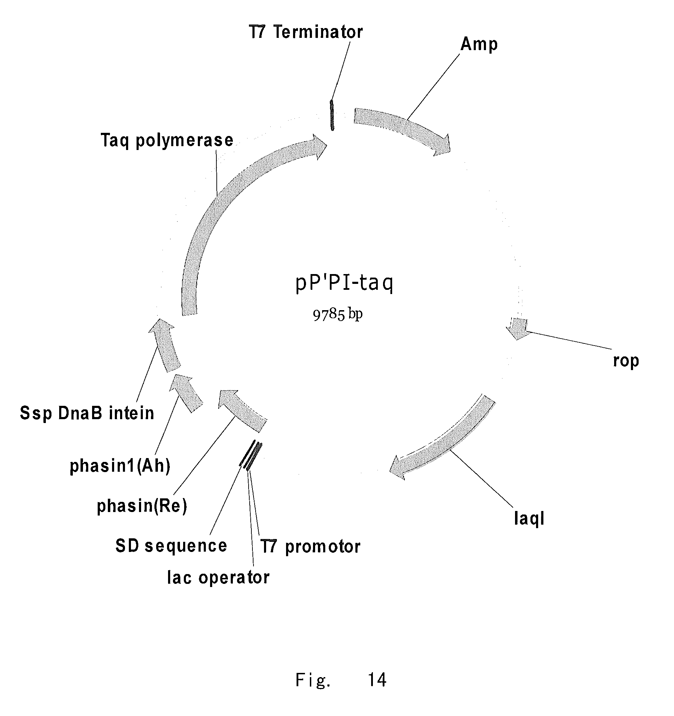

### I. Construction of the fusion expression plasmid pPI-taq (Figure 14), for expressing the fusion protein phasin-intein-taq

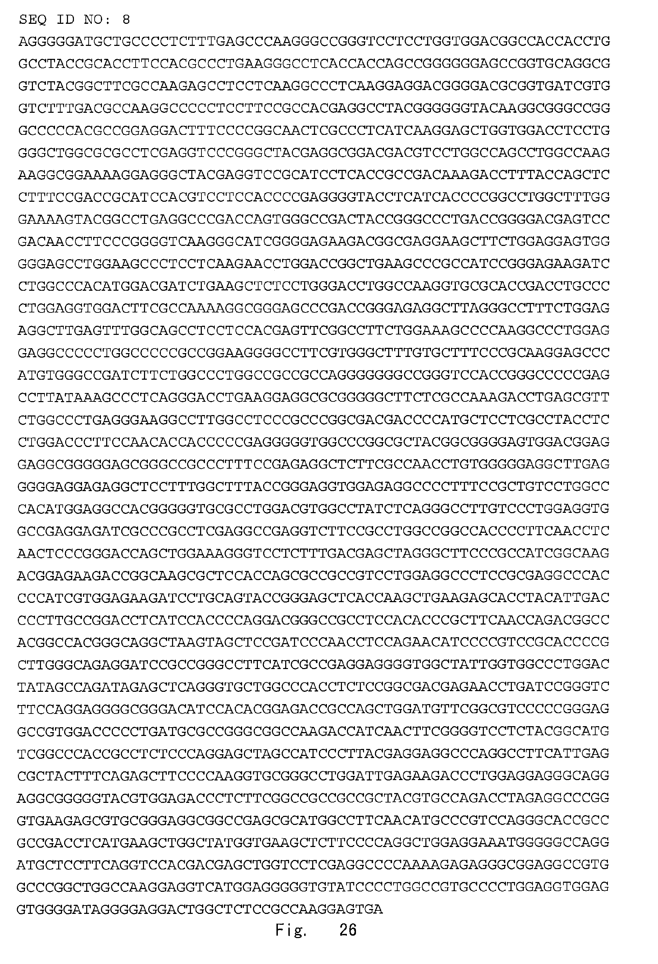

The Taq polymerase gene (SEQ ID No: 8, see Figure 26) obtained through PCR was inserted into the *Bsr*GI and *Bam*HI sites of the plasmid pPI-EGFP. PCR conditions were: 94°C for 3 minutes; 30 cycles of (94°C for 30s, 55°C for 30s and 72°C for 2 minutes and 30 seconds); 72°C for 5 minutes and HOLD at 4°C). The taq gene contains BamHI enzyme cleavage site, and therefore, *Bgl*II (an isocaudarner of *Bam*HI) was used in place of *Bam*HI for designing primers. See Table 2 for the used primers.

### II. Expression and purification steps were the same as Steps II to V in Example 1. The used hydrophobic granules were made from polyethylene (PE).

Results showed that the target protein taq DNA polymerase was obtained in a high purity.

### Example 8 Preparation of a fusion protein of granule associated protein Phasin, autolytic protein Intein and the target protein maltose binding protein (MBP) as well as the purification of MBP

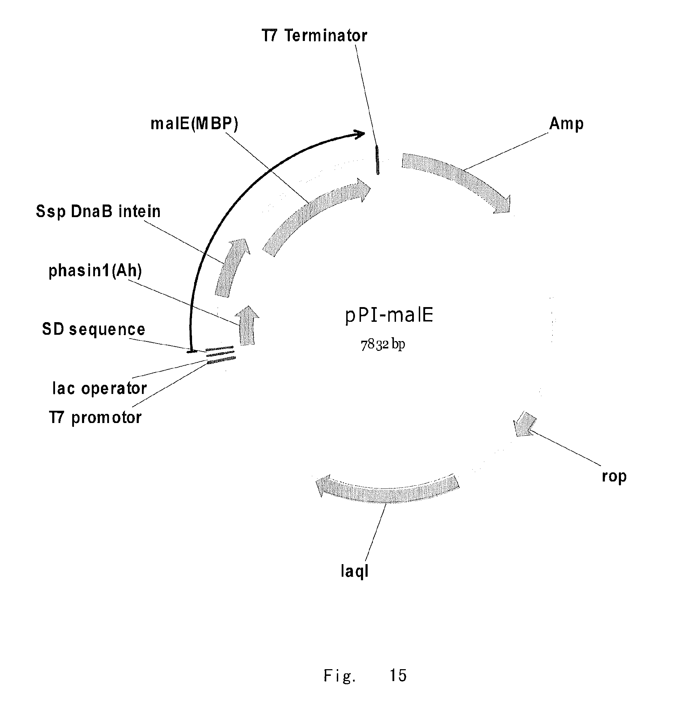

### I. Construction of the fusion expression plasmid pPI-malE (Figure 15), for expressing the fusion protein phasin-intein-MBP

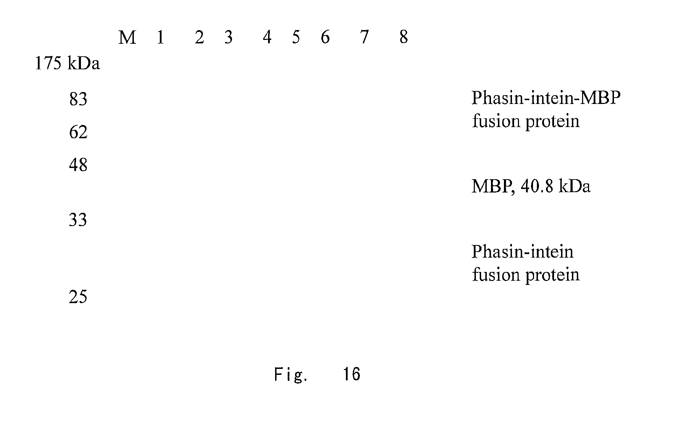

The malE gene (encoding MBP protein, SEQ ID No: 9, see Figure 25) was obtained by PCR (PCR conditions: 94°C for 3 minutes; 30 cycles of (94°C for 30s, 55°C for 30s and 72°C for 1 minute and 10 seconds); 72°C for 5 minutes and holding at 4°C), using pMAL-C2x as the template. Then the malE gene was inserted into the *Bsr*GI and *Bam*HI sites of the plasmid pPI-EGFP. See Table 2 for the used primers. See Figure 16 for the SDS-PAGE electrophoresis pattern.

### II. Expression and purification steps were the same as Steps II to V in Example 1. The used hydrophobic granules were made from PHBHHx.

Results showed that the target protein MBP was obtained in a high purity.

### Example 9 Preparation of a fusion protein of granule associated protein Phasin, autolytic protein Intein and glutathione S-transferase (GST) as well as the purification of GST

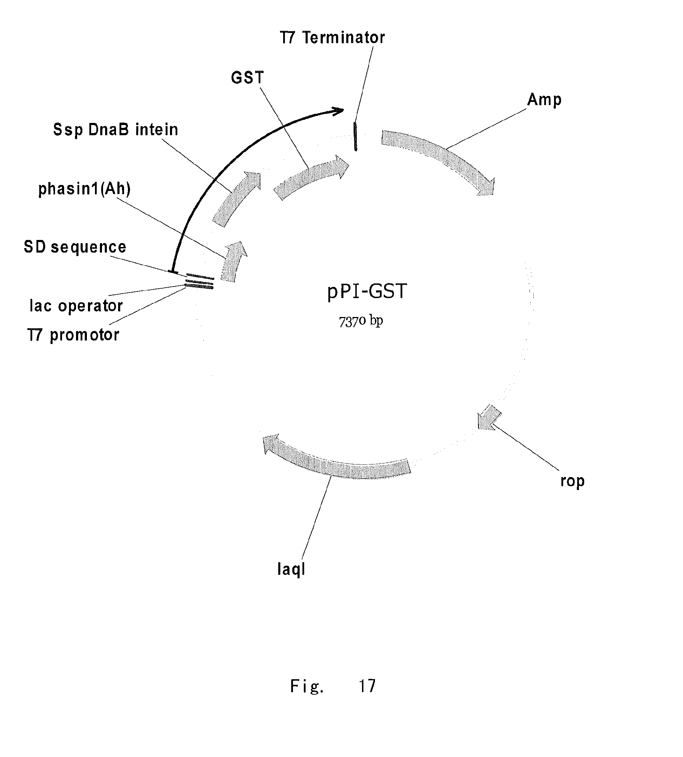

### I. Construction of the fusion expression plasmid pPI-GST (Figure 17), for expressing the fusion protein phasin-intein-GST

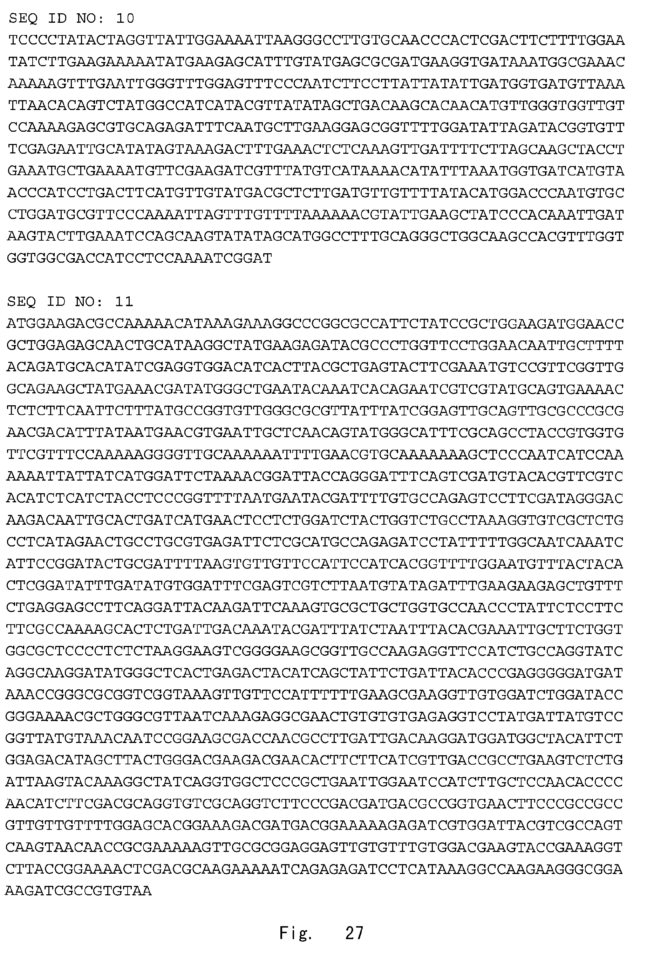

The GST gene (SEQ ID No: 10, see Figure 27) was obtained by PCR (PCR conditions: 94°C for 3 minutes; 30 cycles of (94°C for 30s, 55°C for 30s and 72°C for 40s); 72°C for 5 minutes and holding at 4°C), using pGEX-4T3 as the template. Then the GST gene was inserted into the *Bsr*GI and *Bam*HI sites of the plasmid pPI-EGFP. See Table 2 for the used primers.

### II. Expression and purification steps were the same as Steps II to V in Example 1. The used hydrophobic granules were made from PHB.

Results showed that the target protein GST was obtained in a high purity.

### Example 10 Preparation of a fusion protein of granule associated protein Phasin, autolytic protein Intein and luciferase as well as the purification of luciferase

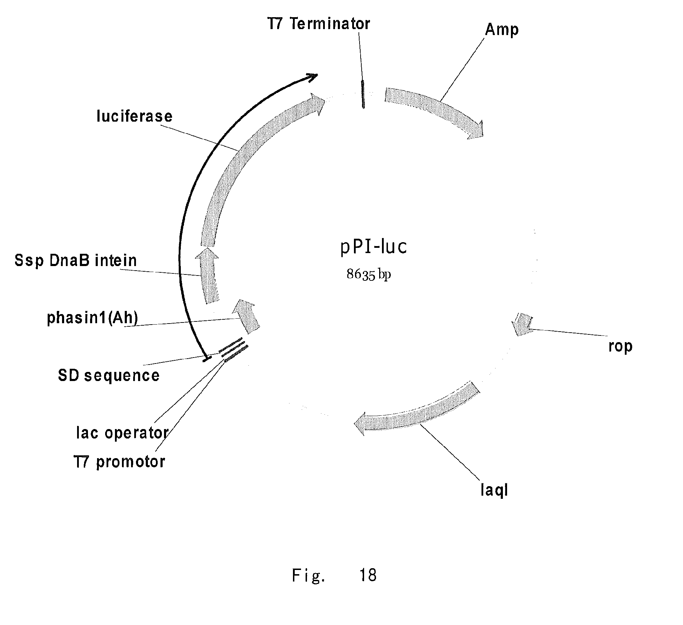

### I. Construction of the fusion expression plasmid pPI-luc (Figure 18), for expressing the fusion protein phasin-intein-luciferase

The Luc gene (SEQ ID No: 11, see Figure 27) is the luc gene part cleaved from the plasmid pGL-promoter by *Nco*I and *Bam*HI double enzyme cleavage. Then the luc gene was inserted into the corresponding site of pPI-EGFP.

### II. Expression and purification steps were the same as Steps II to V in Example 1. The used hydrophobic granules were made from polystyrene (PS).

Results showed that the target protein luciferase was obtained in a high purity.

### Example 11 Preparation of a fusion protein of PHA synthetase PhaC, autolytic protein Intein and green fluorescent protein EGFP as well as the purification of EGFP

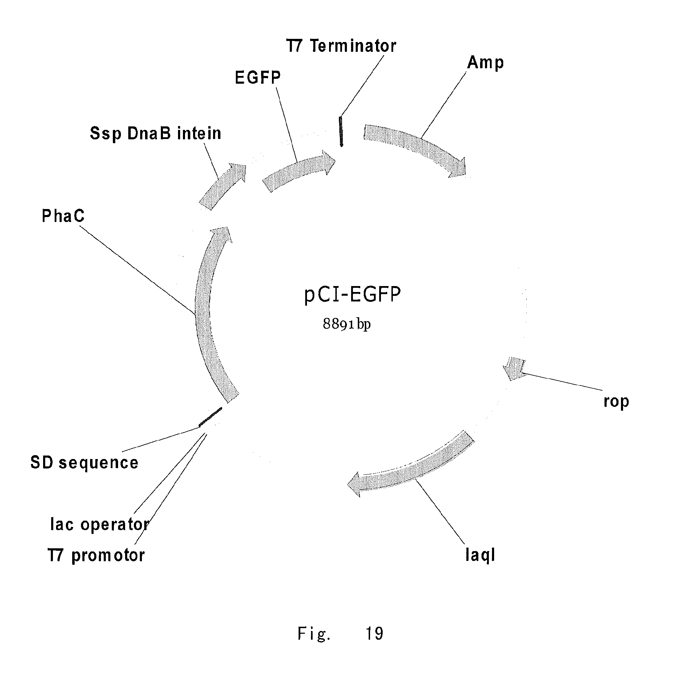

### I. Construction of the fusion expression plasmid pCI-EGFP (Figure 19), for expressing the fusion protein PhaC-intein-EGFP

The *phaC* gene (SEQ ID No: 4) was obtained by PCR (PCR conditions: 94°C for 3 minutes; 30 cycles of (94°C for 30s, 55°C for 30s and 72°C for 1 minute and 50 seconds); 72°C for 5 minutes and holding at 4°C), using the genome of *Ralstonia eutropha* H16 as the template. Then the gene was inserted into the *Bgl*II and *Bam*HI sites of the plasmid pRI-EGFP. The phaC gene contains *Bgl*II enzyme cleavage site, and therefore, *Bam*HI (an isocaudarner of *Bgl*II) was used in place *of Bgl*II for designing primers. See Table 2 for the used primers.

### II. Expression and purification steps were the same as Steps II to V in Example 1. The used hydrophobic granules were made from polypropylene (PP).

Results showed that the target protein EGFP was obtained in a high purity.

### Example 12 Preparation of a fusion protein of three granule associated proteins Phasin, autolytic protein Intein and green fluorescent protein EGFP as well as the purification of EGFP

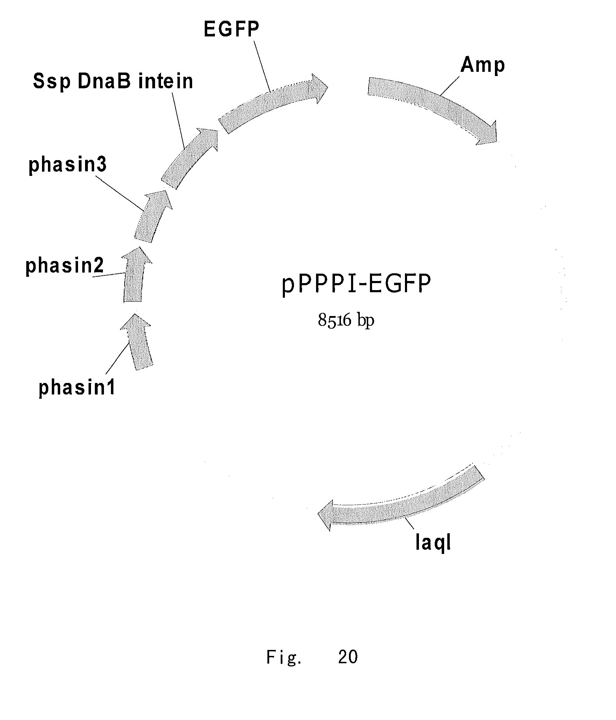

### I. Construction of the fusion expression plasmid pPPPI-EGFP (Figure 20), for expressing the fusion protein phasin-phasin-phasin-intein-EGFP

Modification was made on the basis of pRI-EGFP. Firstly, the *phaP*2 (*phaP*+linker) fragment was inserted between the *Bgl*II*lHind*III sites. Then *phaP*1 (*phaP+*linker) was inserted into the *Bgl*II site, and *phaP*3 (*phaP*) was inserted into the HindIII site. *phaP*1*, phaP*2 and *phaP*3*,* the sizes of which were similar, were obtained by PCR (PCR conditions were same: 94°C for 3 minutes; 30 cycles of (94°C for 30s, 55°C for 30s and 72°C for 25s); 72°C for 5 minutes and holding at 4°C), using the plasmid pPI-EGFP as the template. See Table 2 for the used primers.

### II. Expression and purification steps were the same as Steps II to V in Example 1. The used hydrophobic granules were made from polymethylmethacrylate (PMMA).

Results showed that the target protein EGFP was obtained in a high purity.

### Example 13 Preparation of a fusion protein of granule associated protein Phasin, substrate binding domain of PHA depolymerase (ZSBD), autolytic protein Intein and green fluorescent protein EGFP as well as the purification of EGFP

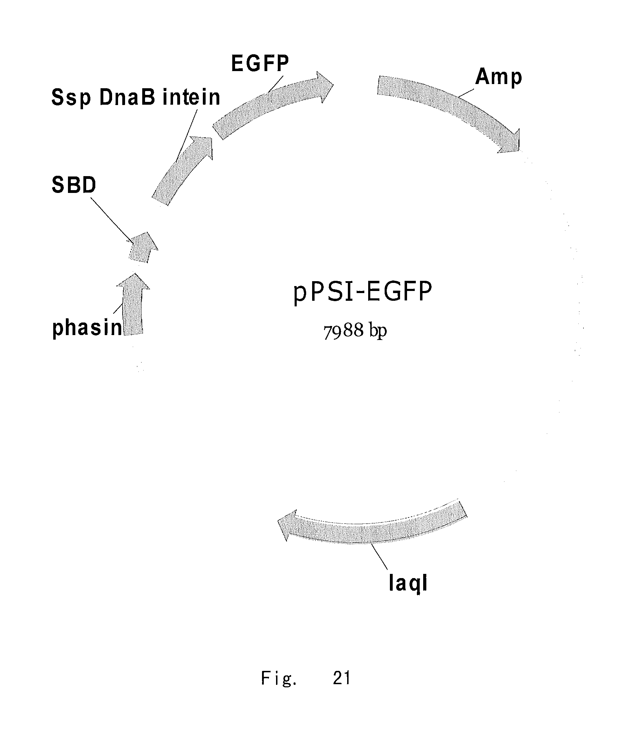

### I. Construction of the fusion expression plasmid pPSI-EGFP (Figure 21), for expressing the fusion protein phasin-ZSBD-intein-EGFP

Modification was made on the basis of pRI-EGFP. Firstly, the *phaP* fragment (*pha*P+linker) was inserted between the *Bgl*II*lHind*III sites. Then ZSBD was inserted into the *Hind*III site. The *phaP* gene was obtained by PCR (PCR conditions: 94°C for 3 minutes; 30 cycles of (94°C for 30s, 55°C for 30s and 72°C for 25s); 72°C for 5 minutes and holding at 4°C), using the plasmid pPI-EGFP as the template. The SBD gene was obtained by PCR (PCR conditions: 94°C for 3 minutes; 30 cycles of (94°C for 30s, 55°C for 30s and 72°C for 20s); 72°C for 5 minutes and holding at 4°C), using the plasmid pRI-EGFP as the template. See Table 2 for the used primers.

### II. Expression and purification steps were the same as Steps II to V in Example 1. The used hydrophobic granules were made from polyvinyl chloride (PVC).

Results showed that the target protein EGFP was obtained in a high purity.

All the references, including patent documents, scientific articles and publications, are entirely incorporated herein by reference.

It is to be understood that variations and modifications can be made by a skilled artisan in the forms and details of the present invention without departing from the spirit and scope of the present invention. All the variations and modifications are intended to be within the scope of the present invention.

**Table 1: Strains and plasmids**

| Strains and plasmids | Relevant features | Origins or references |
|---|---|---|
| Strains | | |
| *E. coli* XL1-Blue | recA1 endA1 gyrA96 thi-1 hsdR17 | Stratagene |
| | supE44 relA1 lac [F' proAB lacIqZ | |
| | M15 Tn10 (Tetr)] | |
| *E. coli* S17-1 | recA pro hsdR RP4-2-Tc::Mu-Km:: Tn7 | ATCC NO. 47055 |
| *E. coli* BL21 (DE3) | E. coli B F- dcm ompT hsdS(rB- mB-) gal λ(DE3) | Stratagene |
| *Aeromonas hydrophila* 4AK4 | | Deposited in Microbiology Lab, Tsinghua University |
| *Ralstonia eutropha* H16 | | Deposited in Microbiology Lab, Tsinghua University |
| *Pichia pastoris* GS 115 | | Invitrogen |
| | | |
| Plasmids | | |
| pTWIN1 | a gene containing Ssp DnaB mini intein and Mxe GyrA mini intein | New England Biolabs, NEB |
| pGEX-4T3 | a gene containing glutathione (GST) | GE healthcare life sciences |
| pGL-promotor | a gene containing luciferase | Promega |
| pMAL-c2x | a gene containing maltose binding protein (MBP) | Biolabs, NEB |
| pGAPZaA | yeast expression plasmid | Invitrogen |

**Table 2: Primers required for constructing the fusion expression plasmid**

| Plasmid | Target fragment | Primer Designation | Primers |
|---|---|---|---|
| pPI-EGFP | EGFP | E1 | TCCTCGAGGGCTCTTCCATGGTGAGCAAGGG CGAGG |
| | | E2 | CCGGATCCTTACTTGTACAGCTCGTCCATGCC |
| | PTWIN1 excluding CBD1 | T1 | AACAACGGTAACAACGGTC |
| | | T2 | ATGTATATCTCCTTCTTAAAGTTAAAC |
| | *phaP*1 | P1 | ATGAATATGGACGTGATCAAGAGCTTTACC |
| | | P2 | GGCCTTGCCCGTGCTCTTC |
| pP'PI-EGFP | *phaP*2 | P3 | TACATATGAATATGGACGTGATCAAGAGCTT TACCG |
| | | P4 | TACATATGTCCGGACTCGCGCAGTTCGAG |
| pPI-lacZ | lacZ | Z1 | TCATTGTACACAACACCATGATTACGGATTC ACTGG |
| | | Z2 | AACGGATCCTTATTTTTGACACCAGACCAAC TG |
| pPI-phaC | *phaC* | C1 | TCATTGTACACAACGCGACCGGCAAAGGCG C |
| | | C2 | AACGGATCCTCATGCCTTGGCTTTGACGTAT CG |
| | pPI-EGFP excluding | IE1 | GAAGATCTTATATCTCCTTCTTAAAGTTAAA C |
| pRI-EGFP | *phaP* | IE2 | CCCAAGCTTAACAACGGTAACAACGGTCTCG |
| | *phaR* | R1 | GAAGATCTATGGCCACGACCAAAAAAG |
| | | R2 | CCCAAGCTTTTACTTCTTGTCCGGCTGG |
| pSBDI-EGFP | ZSBD | S1 | CCCAAGCTTATGGTCGAGGGCGATGCGGAT |
| | | S2 | GGGGTACCCCTGGTGGCCGAGGCCT |
| | pPI-EGFP | SIE1 | GGGGTACCAACAACGGTAACAACGGTCT |
| | excluding *phaP* | SIE2 | CCCAAGCTTATCTCCTTCTTAAAGTTAAAGT TAAA |
| | ORM1 | O1 | AGTATGCATTGTCCTGGGTTCTTACAGTCCTG |
| | | 02 | ATGGTACCGGATTCCCCCTCCTCCTGTTTC |
| pSOIP | *phaP* | P1 | ATGGTACCCATATGATCCTCACCCCGGA |
| | | P2 | AGGTCTAGATTAGGCAGCCGTCGTCTTCTTTG |
| | Mth RIR1 | In1 | ATGGTACCTGCGTATCCGGTGACACCAT |
| | Intein | In2 | ATGGTACCTGCGTGTACAATGAAGCCAT |
| pPI-taq | Taq | T1 | TCATTGTACACAACAGGGGGATGCTGCCCCTCTT |
| | | T2 | AACAGATCTTCACTCCTTGGCGGAGAGCC |
| pPI-malE | malE | M1 | TCATTGTACACAACATGAAAATCGAAGAAG GTAAACTGG |
| | | M2 | AACGGATCCTGCAGTTATCGAGCTCGAATTA GTCTGCG |
| pPI-GST | GST | GST1 | TCATTGTACACAACTCCCCTATACTAGGTTA TTGGA |
| | | GST2 | AACGGATCCTCAATCCGATTTTGGAGGATG |
| pCI-EGFP | *phaC* | C1 | ATAGGATCCATGGCGACCGGCAAAGGCGC |
| | | C2 | ACCAAGCTTTGCCTTGGCTTTGACGTATCGCCC |
| | *phaP*2 | P1F | GAAGATCTATGAATATGGACGTGATCAAGA GCTTTACC |
| | | P1R | CCCAAGCTTTCCGGACTCGCGCAGTTCG |
| pPPPI-EGFP | *phaP*1 | P1F | GAAGATCTATGAATATGGACGTGATCAAGA GCTTTACC |
| | | P2R | GAAGATCTTCCGGACTCGCGCAGTTCG |
| | *phaP*3 | P3F | CCCAAGCTTATGAATATGGACGTGATCAAGA GCTTTACC |
| | | P3R | CCCAAGCTTGGCCTTGCCCGTGCTCTTCT |
| pPSI-EGFP | *phaP* | P1F | GAAGATCTATGAATATGGACGTGATCAAGA GCTTTACC |
| | | P2R | CCCAAGCTTTCCGGACTCGCGCAGTTCG |
| | ZSBD | SF | CCCAAGCTTATGGTCGAGGGCGATGCGGAT |
| | | SR | CCCAAGCTTCCTGGTGGCCGAGGCCT |

## Claims

1. A method for purifying a recombinant target protein, comprising the steps of:
(1) recombinantly expressing a fusion protein in a host cell, wherein said fusion protein comprises a target protein domain, an autolytic Intein and one or multiple hydrophobic granule binding domains, and wherein said autolytic Intein is located between said target protein domain and said one or multiple hydrophobic granule binding domains;
(2) releasing said fusion protein from said host cell, to obtain a solution containing said fusion protein;
(3) adding hydrophobic granules to said solution and incubating the solution under a condition which allows the binding of said fusion protein with said hydrophobic granules;
(4) collecting the incubated hydrophobic granules from said solution;
(5) adding a lysis solution to said hydrophobic granules and treating said hydrophobic granules under a condition which allows the autolytic Intein in said fusion protein to disrupt; and
(6) removing said hydrophobic granules, to obtain a solution containing said target protein which is substantially purified.

2. The method of Claim 1, further comprising an optional step of washing said hydrophobic granules after step (4).

3. The method of Claim 1, wherein said host cell is derived from a prokaryote.

4. The method of Claim 3, wherein said prokaryote is selected from the group consisting of *Escherichia coli, Ralstonia eutropha, Pseudomonas spp.* and *Bacillus spp..*

5. The method of Claim 1, wherein said host cell is derived from a eukaryote.

6. The method of Claim 5, wherein said eukaryote is selected from the group consisting of *Saccharomyces cerevisiae* and *Pichia pastoris.*

7. The method of Claim 1, wherein said autolytic Intein is selected from the group consisting of Ssp DnaB mini-intein, Mxe GyrA intein, Mth RIR1 intein and Sce VMA1 intein.

8. The method of Claim 1, wherein said hydrophobic granule binding domain is selected from the group consisting of Phasin, PhaZ, PhaR, PhaC, lipase, ZSBD and RSBD.

9. The method of Claim 1, wherein the number of said hydrophobic granule binding domain is one, two or three.

10. The method of Claim 1, wherein said multiple hydrophobic granule binding domains are not exactly the same.

11. The method of Claim 1, wherein said one or multiple hydrophobic granule binding domains are selected from the group consisting of Phasin-Phasin, Phasin-Phasin-Phasin, ZSBD-ZSBD-ZSBD, RSBD-RSBD-RSBD, PhaC-PhaC-PhaC, Phasin-ZSBD, ZSBD-RSBD and Phasin-ZSBD-RSBD.

12. The method of Claim 1, wherein said multiple hydrophobic granule binding domains are linked via a linker.

13. The method of Claim 1, wherein said autolytic Intein is linked to said one or multiple hydrophobic granule binding domains via a linker.

14. The method of Claim 1, wherein said autolytic Intein is directly linked to said target protein domain.

15. The method of Claim 1, wherein said target protein domain, said autolytic Intein and said at least one hydrophobic granule binding domain are within the same reading frame.

16. The method of Claim 1, wherein said recombinant expression is carried out by introducing an expression vector comprising a nucleic acid encoding said fusion protein into said host cell followed by culturing said host cell.

17. The method of Claim 1, wherein said hydrophobic granules are formed from a hydrophobic polymer material selected from the group consisting of polyethylene, polyvinyl alcohol, polystyrene, polylactic acid, polycaprolactone, polypropylene, polymethyl methacrylate and polyvinyl chloride.

18. The method of Claim 1, wherein said hydrophobic granules are formed from a hydrophobic PHA material selected from the group consisting of polyhydroxybutyrate, co-polymer of hydroxybutric acid and hydroxyvaleric acid, co-polymer of hydroxybutric acid and hydroxyhexanoic acid, polyhydroxycaprylate, polyhydroxyenanthate, polyhydroxydecanoate, co-polymer of hydroxybutric acid and hydroxycaprylic acid, co-polymer of hydroxybutric acid, hydroxyvaleric acid and hydroxyhexanoic acid.

19. The method of Claim 1, wherein the collection and/or isolation of said hydrophobic granules are carried out by centrifugation and/or filtration.

20. The method of Claim 1, wherein a magnetic granule is enwraped within said hydrophobic granules.

21. The method of Claim 1, wherein said hydrophobic granules comprise a core consisting of super-paramagnetic powder, which core is coated by a hydrophobic material.

22. The method of Claim 21, wherein said super-paramagnetic powder is Fe₃O₄ magnetic powder.

23. The method of Claim 20, wherein a magnet is used to collect and/or isolate said hydrophobic granules.

24. A kit for purifying a recombinant target protein, comprising:
an expression vector for recombinantly expressing a fusion protein in a host cell, wherein said fusion protein comprises a target protein domain, an autolytic Intein and one or multiple hydrophobic granule binding domains, and wherein the autolytic Intein is located between said target protein domain and said one or multiple hydrophobic granule binding domains; and
hydrophobic granules.

25. The kit of Claim 24, wherein said hydrophobic granules are formed from a hydrophobic polymer material selected from the group consisting of polyethylene, polyvinyl alcohol, polystyrene, polylactic acid, polycaprolactone, polypropylene, polymethyl methacrylate and polyvinyl chloride.

26. The kit of Claim 24, wherein said hydrophobic granules are formed from a hydrophobic PHA material selected from the group consisting of polyhydroxybutyrate, co-polymer of hydroxybutric acid and hydroxyvaleric acid, co-polymer of hydroxybutric acid and hydroxyhexanoic acid, polyhydroxycaprylate, polyhydroxyenanthate, polyhydroxydecanoate, co-polymer of hydroxybutric acid and hydroxycaprylic acid, co-polymer of hydroxybutric acid, hydroxyvaleric acid and hydroxyhexanoic acid.

27. The kit of Claim 24, wherein a magnetic granule is enwraped within said hydrophobic granules.

28. The kit of Claim 24, wherein said hydrophobic granules comprise a core consisting of super-paramagnetic powder, which core is coated by a hydrophobic material.

29. The kit of Claim 28, wherein said super-paramagnetic powder is Fe₃O₄ magnetic powder.

30. The kit of Claim 24, further comprising a magnet.
